# EUROPEAN PATENT SPECIFICATION

(11) **EP 1 796 558 B1**
(45) Date of publication and mention of the grant of the patent: **03.12.2008**
(21) Application number: 04787594.3
(22) Date of filing: 16.09.2004
(51) Int. Cl.: A61B 17/17

(54) **ALIGNING DEVICE FOR CORRECTLY INSERTING SCREWS INTO HOLES OF ENDOMEDULLARY PROSTHESES**
AUSRICHTEVORRICHTUNG FÜR DIE KORREKTE EINFÜHRUNG VON SCHRAUBEN IN DIE LÖCHER VON ENDOMEDULLÄREN PROTHESEN
DISPOSITIF D'ALIGNEMENT POUR INSERER CORRECTEMENT DES VIS DANS DES TROUS DE PROTHESES ENDOMEDULLAIRES

(43) Date of publication of application: 20.06.2007
(73) Proprietor: Alfa Plastic S.R.L., I-42010 Rio Saliceto (IT)
(72) Inventor: FERRARI, Luigi, I-41049 Sassuolo (IT); SACCHI, Edo, I-42015 Correggio (IT)
(74) Representative: Corradini, Corrado
(86) International application number: PCT/IT2004/000502
(87) International publication number: WO 2006/030460

(56) References cited:
- EP-A- 0 465 436
- US-A- 4 803 976
- US-B1- 6 214 013

## Description

### TECHNICAL FIELD

The present invention relates to an aligning device for correctly inserting screws into through holes provided transversely in an endomedullary prosthesis, and the relative alignment method

### PRIOR ART

In the case of a comminuted bone fracture, a prosthesis, commonly known as a nail, has to be inserted and locked into the bone through which it passes.

The intramedullary nail is locked by inserting two screws percutaneously and passing them through holes provided in the proximal and/or distal end of the nail.

Inserting the screws through both ends of the nail fixes the proximal and distal fragments, preventing them from sliding along the nail.

This type of synthesis is defined as "static".

Alternatively the screws can be inserted through only one end of the nail, to fix only one of the larger fragments.

This type of synthesis is defined as "dynamic".

Before nailing, generally of closed roof type, pre-operative radiography must be carried out on the healthy bone to determine the correct nail size, the degree of boring and the final length of the nail in seriously comminuted fractures.

In femur fractures, the nail length must be such that its proximal end lies at the apex of the greater trochanter and its distal end between the upper pole of the patella and the distal epiphyseal cicatrix of the femur.

The intramedullary nail is inserted into the centre of the direct continuation of the medullary channel in the anteroposterior and laterolateral projections using a handle suitably associated, in a removable manner, with the proximal end of the nail.

When inserted, the nail forms an angle of 5°-10° open laterally to the saggital plane of the femur, such as to be aligned with the femoral axis. On termination of nail insertion it is locked by inserting screws.

To enable the screws to be inserted, a cutter is generally used applied to a drill, to drill the femur by passing through the holes in the nail.

To ensure that the cutter is correctly centered on the holes present in the nail, the axis on which the cutter operates and the axis of the corresponding hole in the nail must be aligned and consequently coinciding.

This correct centering can be achieved using an aligning device comprising a support associated with the nail handle and carrying a guide bush within which the cutter slides as an exact fit and which is aligned with the corresponding hole before inserting the nail into the femur, recording the position so achieved.

After recording the position the aligning device is removed to allow easier nail insertion, and then is again associated with the handle by which the nail is thrust.

If the nail does not undergo any bending, the alignment between the bush and the corresponding hole is correct, as the holes have not undergone any spatial displacement from their position recorded prior to insertion. Instead, as the nail inserted into the medullary bone cavity undergoes a certain bending to comply with the natural shape of the endomedullary path and as this bending is more marked the further from the proximal end of the nail with which the insertion handle is associated, a misalignment often occurs between the bush and the corresponding hole, particularly for the distal holes.

In this respect, while the orientation and spatial disposition of the proximal holes remains substantially unchanged, so enabling centering of the proximal holes to be easily achieved as bending there is irrelevant, the spatial disposition of the distal holes can undergo a relatively substantial shift because of the albeit slight bending undergone by the nail at the distal end.

In practice, centering of the proximal holes is relatively simple to achieve because nail bending at that distance is irrelevant, whereas this is not the case with the distal holes.

Consequently, the spatial disposition of the guide bush has to be corrected to bring it into correct alignment with the corresponding hole which has shifted.

Radiography is generally used to verify perfect alignment between the hole and the corresponding bush.

This technique enables the extent of misalignment to be ascertained between the hole in the nail and the guide bush which is rigidly connected to the head of the nail by the handle.

Even though from the images it may appear that the cutter guide bush and the corresponding hole are aligned, they are in reality often misaligned.

In this respect, alignment between the guide bush of traditional aligning devices and the corresponding hole cannot be ascertained accurately by radiography or any other detection method of the known art because the resultant images are not sufficiently accurate to allow detection of slight displacements from perfect alignment, with a consequent substantial error in the subsequent drilling with the cutter.

This means that on the one hand a multiplicity of radiographs have to be taken, with in the limit a continuous radiograph during drilling, in order to follow the cutter path through the bone and continuously verify alignment with the corresponding hole, while on the other hand, in the case of error, drilling has to be done at several points in order to correctly "centre" the corresponding hole.

In this manner not only is the time required to achieve complete locking of the nail to the bone increased, but there is the risk of further damaging the already fragile bone by the continuous attempts to centre the hole with the cutter.

US 6 214 013 shows an aligning device and a method for correctly inserting a screw into a respective through hole of an endomedullary prosthesis implanted in a bone tissue. The aligning device has a support associated with the prosthesis and guiding means defining an intervention aiming line for the perforation means. The guide means are detectable by selective detection means. The device further comprise orientation means for spatially orientating the support means and the support means carry also a target with sight detectable by the detection means. However the target with sight is not aligned in position with the aiming line and the through hole and the hole is not interposed between the guide means and the target, with its axis disposed on the aiming line.

There is therefore a strongly felt requirement for an aligning device for correctly inserting screws into the holes of a prosthesis which enables the detection means, such as radiographic means, to determine exactly and without error when perfect hole centering has been achieved, so preventing further damage to the bone concerned and/or lengthening of the time required to completely lock the nail.

### DISCLOSURE OF THE INVENTION

An object of the present invention is to provide an aligning device for correctly inserting screws into the holes of an endomedullary prosthesis having structural and functional characteristics such as to satisfy the aforesaid requirements while at the same time obviating the stated drawbacks of the known art.

These objects are attained by an aligning device for correctly inserting screws into the holes of a prosthesis in accordance with claim 1.

The dependent claims define preferred and particularly advantageous embodiments of the device of the invention.

### BRIEF DESCRIPTION OF THE DRAWINGS

Further characteristics and advantages of the invention will be apparent on reading the ensuing description given by way of non-limiting example with reference to the figures of the accompanying drawings, in which:

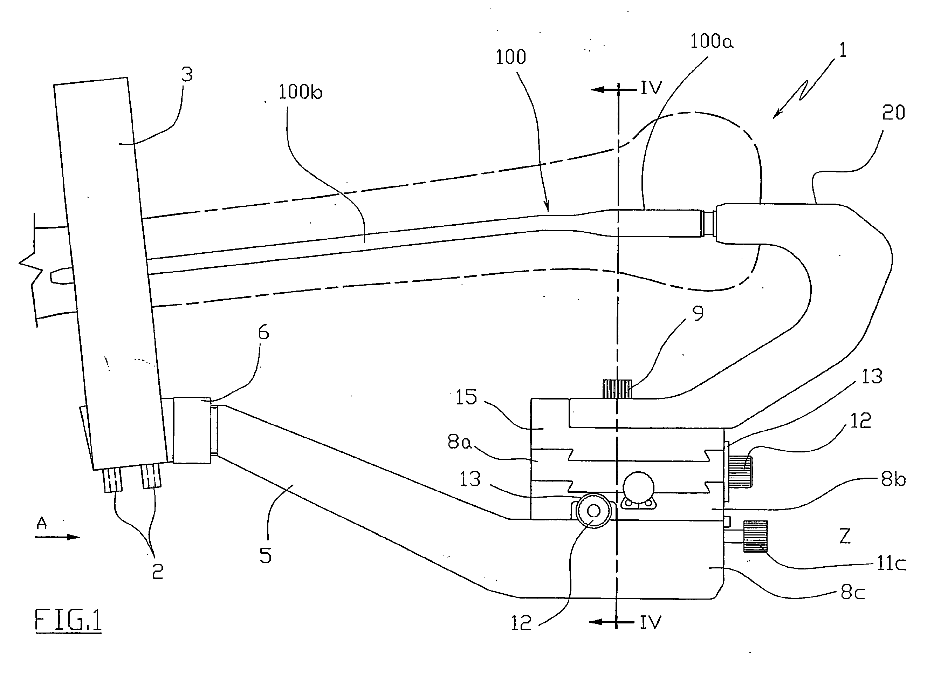

Figure 1 is a side view of an aligning device in accordance with the present invention;
Figure 2 is a view in the direction of the arrow A of Figure 1;
Figure 3 is the section on the broken line III-III of Figure 2;

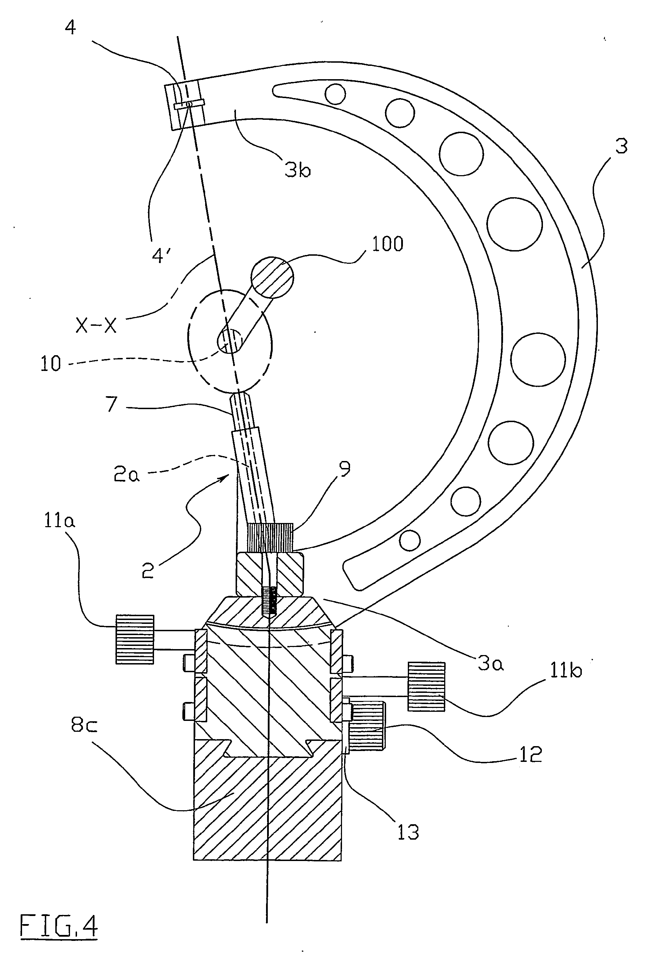

Figure 4 is the section on the line IV-IV of Figure 1.

### BEST MODE FOR CARRYING OUT THE INVENTION

With reference to said figures, the reference numeral 1 indicates overall an aligning device of the present invention for inserting a screw into a respective through hole 10 of a prosthesis 100, generally known as an intramedullary nail, inserted into a bone tissue.

Explicit reference will be made hereinafter to a femoral prosthesis and to the insertion of screws through two so-called distal holes, but without being limited to this application.

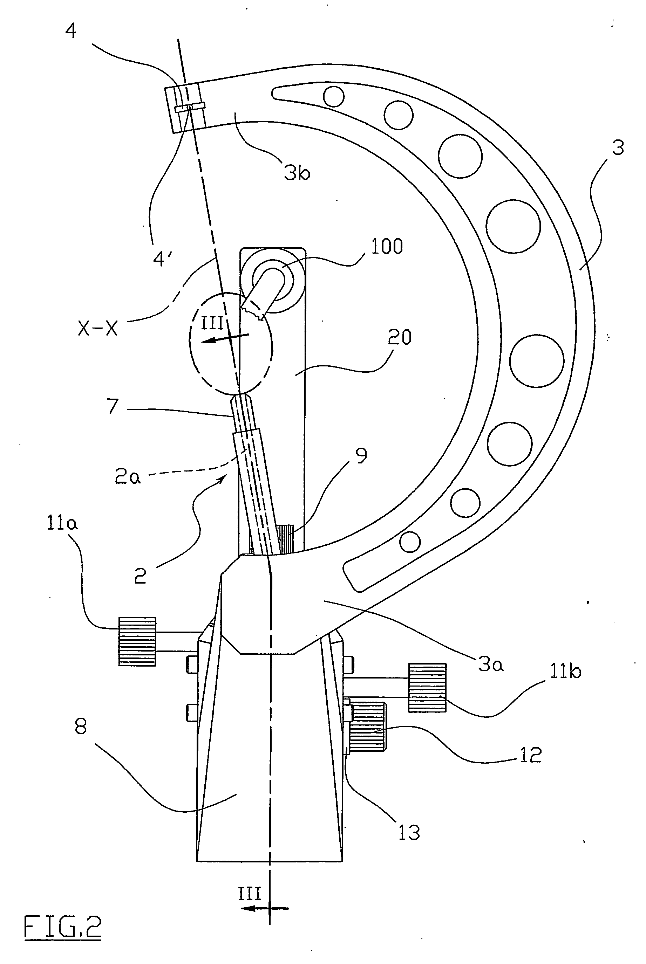

Said device 1 comprises essentially (Figure 2) support means 3 associated with said nail 100 and carrying respectively guide means 2 to define an aiming intervention line X-X for bone perforation means and a target 4 with sight 4' aligned with said aiming line and intended to be positioned facing the hole 10 on the opposite side to that on which the guide means 2 are disposed during bone perforation, together with orientation means 5, 8 for spatially orientating said support means 3. According to the preferred embodiment of the present invention, the support means 3 are in the form of an arcuate element to which at its first end 3a the guide means 2 and at its second end 3b the target 4 are fixed. In the illustrated example, said guide means 2 are in the form of a pair of bushes 2a extending along two parallel aiming lines X-X.

Although reference is made to a pair of bushes 2a, a single bush or more than two bushes can be used.

In the illustrated example, the arcuate element 3 is fixed at its first end 3a to a bracket 5 by screwing a locking nut 6 able to prevent sliding and rotation of the arcuate element 3 and consequently of the bushes 2a and of the target 4.

The bracket 5 is fixed at its opposite end to the proximal end of the nail 100. According to the invention, the target 4 comprises two punctiform sights 4', each positioned such as to be always aligned with the respective aiming line X-X of the pair of bushes 2a, i.e. with the direction in which the perforation means travel.

Essentially, each bush 2a is always correctly aligned with the corresponding sight 4' even following any spatial movements of the arcuate element 3.

The perforation means, such as a cutter, this latter applied to a drill in known manner, can slide within the bushes 2a to reach and traverse the distal holes 10.

In order to perforate with greater precision along an unequivocal direction, further cylindrical guides 7 can be inserted coaxially into the bushes 2a to abut against the femoral bone tissue after incising the thigh, they being shaped to receive the drilling cutters as an exact fit.

During normal use, the arcuate element 3 is positioned about the thigh with the bushes 2a disposed on one side of the holes 10 and the corresponding sights 4' on the opposite side.

Essentially, the holes 10 lie interposed between the bushes 2a and the target sights 4'.

To achieve correct centering of the distal holes 10 of the intramedullary nail 100, the bracket 5 to which the arcuate element 3 is fixed can be orientated relative to the proximal end of the nail 100 by suitable adjustment means 8, preferably able to transmit millimetric movements. Said adjustment means 8 are interposed between the bracket 5 and the end of a U-shaped handle 20 carrying the intramedullary nail 100 associated with its remaining end and prevented from rotating by usual means.

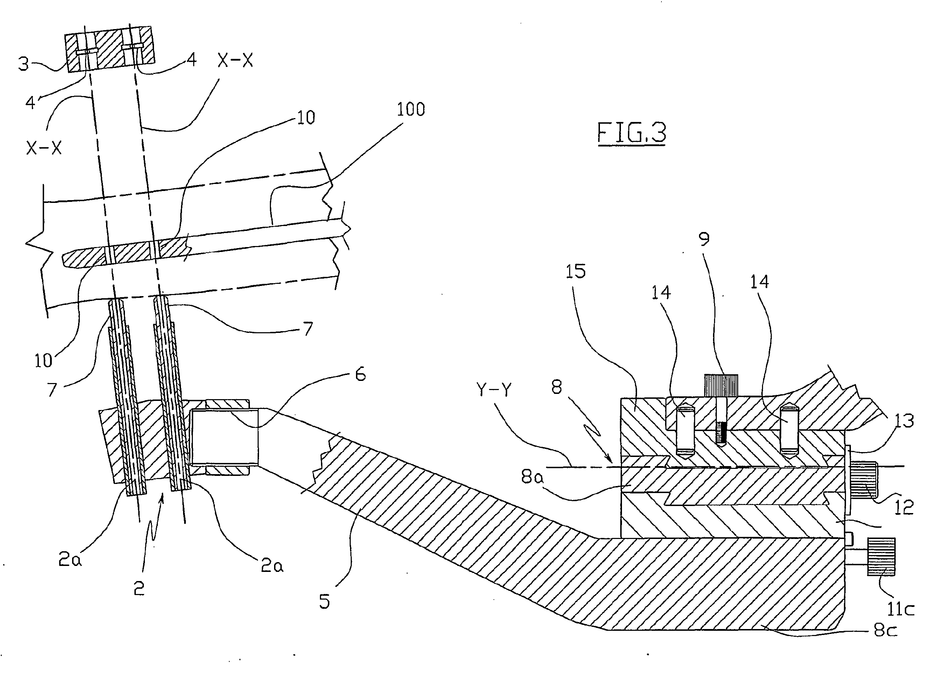

Generally, the nail 100 presents (Figure 3) a first proximal portion 100a associated with the handle 20 and of greater diameter than the prevalent remaining part 100b which presents a 5°-10° deviation from the first portion 100a to follow the shape of the femoral bone.

In the illustrated example, the adjustment means 8 are in the form of three movable elements, an upper 8a, an intermediate 8b and a lower 8c, connected movably together to form a single adjustment block.

The adjustment block is connected by a fixed element 15 to the handle 20, to which it is fixed by a fixing screw 9 with the aid of two centering dowels 14.

The adjustment means 8 enable the bracket 5, and hence its associated arcuate element 3 carrying the bush 2a and the target 4, to be moved spatially.

The movement is adjusted by the micrometric adjustment screws 11a, 11b, 11c which enable the bracket 5 to rotate about a rotation axis Y-Y extending in the example substantially parallel to the axis of the proximal portion 100a of the nail 100 and, respectively, to slide along two mutually perpendicular axes, one of which is parallel to the rotation axis Y-Y.

To prevent any undesirable movement of the bracket 5 after attaining correct alignment between the bushes 2a and holes 10, two locking screws 12 with respective washers 13 are used, suitably located on the adjustment block.

By selective tightening or slackening, said locking screws 12 enable the desired movements to be adjusted, in accordance with the known art.

In operation, having chosen the nail 100 of appropriate length, it is inserted into alignment with the femoral axis in accordance with the known art.

After its insertion with the aid of the handle 20, the stage of locking the nail 100 by inserting screws through the proximal and distal holes 10 begins.

In this latter case, as stated, hole centering is far more difficult to achieve, hence only the insertion of the screws through the distal holes 10 will be described.

The aligning device 1 of the present invention is fixed to the handle 20 by the fixing screw 9 with the adjustment means 8 interposed, while the arcuate element 3 is positioned about the thigh with the holes 10 of the nail 100 interposed between the target 4 and the bushes 2a.

In practice the aligning device 1 is fixed to the handle 10 before inserting the nail 100, the arcuate element 3 being positioned such that the axes of the nail holes 10 are perfectly aligned and hence coinciding with the aiming lines X-X of the corresponding bushes 2a, the position thus obtained being recorded.

When the nail 100 is to be inserted into the femur and the device 1 again fixed to the handle 20 with the arcuate element 3 embracing the thigh, the bushes 2a will already have an orientation close enough to enable perfect centering between the holes 10 and the cutter.

A radiographic examination enables perfect alignment to be determined between the centres of the bushes 2a through which the aiming lines X-X pass coinciding with the axes thereof and the centre of the holes 10, by virtue of the presence of the sights 4' which constructively lie on the corresponding aiming lines X-X.

In this respect, by using materials which show under radiation, such as metals, for constructing the bushes 2a and the target 4 with the sights 4', and considering that the nail 100 shows under radiation, when perfect centering has been achieved the radiography plate will show for each hole 10 a first circle of the bush 2a, a second circle of the hole 10, these being concentric, and a point indicating the sight 4' of the target 4 lying perfectly at the centre of the first two circles.

In contrast, if perfect centering of the holes 10 has not been achieved, at least the point indicating the sight 4' of the target 4 will be off-centre to indicate misalignment between the axis of the hole 10 and the aiming line X-X corresponding to the drilling direction.

To obtain easily readable images the arcuate element 3 should be constructed of a material which does not show under X-rays.

Alternatively, other systems could be used to display correct positioning such as a brightness amplifier.

The target shape is chosen such as to clearly indicate one or more unequivocal points which become positioned perfectly at the centre of the circles visible on the plates when perfect centering has been achieved.

As will be apparent from the description, the aligning device and the alignment method of the present invention satisfy the requirements and overcome the drawbacks stated in the introduction to the present description with reference to the known art.

In this respect said device enables the holes provided in an endomedullary prosthesis to be perfectly centered at the first attempt, so avoiding compromising the bone structure surrounding the holes. Numerous modifications and variants can be devised by an expert of the art to satisfy specific contingent requirements, all lying within the scope of protection of the invention as defined by the following claims.

## Claims

1. An aligning device (1) for correctly inserting a screw into a respective through hole (10) of an endomedullary prosthesis (100) implanted in a bone tissue, comprising support means (3) associated with said prosthesis (100) and carrying guide means (2) defining an intervention aiming line (X-X) for perforation means for said bone tissue, said guide means (2) being detectable by selective detection means, and further comprising orientation means (5, 8) for spatially orientating said support means (3), wherein said support means (3) further carry a target (4) with sight (4') detectable by said detection means, **characterised in that** said target is positioned aligned with said aiming line (X-X), said hole (10) being interposed between said guide means and said target, with its axis disposed on said aiming line (X-X).

2. A device (1) as claimed in claim 1, wherein said support means (3) are associated with said prosthesis (100) by way of said orientation means (8).

3. A device (1) as claimed in claim 1, wherein said guide means (2) comprise at least one guide bush (2a) for slidingly containing perforation means.

4. A device (1) as claimed in claim 3, wherein said support means (3) comprise an arcuate element (3), to a first end (3a) of which said guide means (2) are rigidly fixed, and to the free second end (3b) of which said target (4) is rigidly fixed.

5. A device (1) as claimed in claim 4, wherein said orientation means comprise a movable bracket (5) and adjustment means (8) to orientate it spatially relative to the prosthesis.

6. A device (1) as claimed in claim 5, wherein said arcuate element (3) is associated with an end of said movable bracket (5).

7. A device (1) as claimed in claim 5, wherein said adjustment means (8) enable said bracket (5) to rotate about a rotation axis (Y-Y) and to translate along two mutually perpendicular directions (Y-Y, Z-Z).

8. A device (1) as claimed in claim 5, wherein said adjustment means (8) comprise mutually cooperating movable elements (8a, 8b, 8c) operated by adjustment screws (11a, 11b, 11c).

9. A device (1) as claimed in claim 1, wherein said support means (3) are insensitive to said detection means.

10. A device (1) as claimed in claim 1, wherein said bone tissue is a femur and said through hole (10) is a distal hole.

## Patentansprüche

1. Eine Ausrichtvorrichtung (1) zum korrekten Einführen einer Schraube in eine entsprechende Durchgangsbohrung (10) einer intramedullären Prothese (100), die in ein Knochengewebe implantiert ist, einschließlich Haltevorrichtungen (3), die der Prothese (100) zugeordnet sind, und die Führungsvorrichtungen (2) tragen, welche eine Eingriffsziellinie (X-X) für Perforationsvorrichtungen für das Knochengewebe festlegen, wobei die Führungsvorrichtungen (2) durch selektive Zielerfassungsvorrichtungen erfasst werden können; einschließlich Orientierungsvorrichtungen (5, 8) zur räumlichen Ausrichtung der Haltevorrichtungen (3), wobei die Haltevorrichtungen (3) ein Zielobjekt (4) mit Visier (4') tragen, das von den Zielerfassungsvorrichtungen erfasst werden kann, **dadurch gekennzeichnet, dass** das Zielobjekt auf die Ziellinie (X-X) ausgerichtet ist und sich die Bohrung (10) zwischen den Führungsvorrichtungen und dem Zielobjekt mit der Achse auf der Ziellinie (X-X) befindet.

2. Eine Vorrichtung (1) nach Patentanspruch 1, wobei die Haltevorrichtungen (3) der Prothese (100) durch die Orientierungsvorrichtungen (8) zugeordnet sind.

3. Eine Vorrichtung (1) nach Patentanspruch 1, wobei die Führungsvorrichtungen (2) wenigstens eine Führungsbuchse (2a) beinhalten, die gleitend Perforationsvorrichtungen enthält.

4. Eine Vorrichtung (1) nach Patentanspruch 3, wobei die Haltevorrichtungen (3) ein Bogenelement (3) beinhalten, an dessen erstem Ende (3a) die Führungsvorrichtungen (2) starr angebracht sind und an dessen freiem zweiten Ende (3b) das Zielobjekt starr angebracht ist.

5. Eine Vorrichtung (1) nach Patentanspruch 4, wobei die Orientierungsvorrichtungen eine bewegliche Klammer (5) und Einstellvorrichtungen (8) beinhalten, um sie räumlich in Bezug auf die Prothese auszurichten.

6. Eine Vorrichtung (1) nach Patentanspruch 5, wobei das Bogenelement (3) einem Ende der beweglichen Klammer (5) zugeordnet ist.

7. Eine Vorrichtung (1) nach Patentanspruch 5, wobei die Einstellvorrichtungen (8) die Klammer (5) um eine Drehachse (Y-Y) rotieren und sich auf zwei zueinander senkrechten Richtungen (Y-Y, Z-Z) verschieben lassen.

8. Eine Vorrichtung (1) nach Patentanspruch 5, wobei die Einstellvorrichtungen (8) zusammenwirkende bewegliche Elemente (8a, 8b, 8c) beinhalten, die von Einstellschrauben (11a, 11b, 11c) betrieben werden.

9. Eine Vorrichtung (1) nach Patentanspruch 1, wobei die Haltevorrichtungen (3) unempfindlich auf die Zielerfassungsvorrichtungen sind.

10. Eine Vorrichtung (1) nach Patentanspruch 1, wobei das Knochengewebe ein Oberschenkelknochen ist und die Durchgangsbohrung (10) eine distale Schraubenbohrung ist.

## Revendications

1. Un dispositif d'alignement (1) pour introduire correctement une vis dans le trou passant (10) correspondant d'une prothèse endomédullaire (100) implantée dans un tissu osseux, qui comprend des dispositifs porteurs (3) associés à ladite prothèse (100) et portant des dispositifs de guidage (2) définissant une ligne de visée d'intervention (X-X) de dispositifs de perforation du tissu osseux concerné. Lesdits dispositifs de guidage (2) peuvent être détectés par des moyens de détection sélectifs, ainsi que des moyens d'orientation (5, 8) qui orientent spatialement les dispositifs porteurs (3). Ces derniers portent en outre une cible (4) avec vision (4') détectable par lesdits moyens de détection et placée alignée par rapport à la ligne de visée (X-X). Ledit trou (10) est installé entre les dispositifs de guidage et la cible, avec son axe placé sur la ligne de visée (X-X).

2. Un dispositif (1) selon la revendication 1, où lesdits dispositifs porteurs (3) sont associés avec ladite prothèse (100) par l'intermédiaire desdits moyens d'orientation (8).

3. Un dispositif (1) selon la revendication 1, où lesdits dispositifs de guidage (2) comprennent au moins une douille de guidage (2a) pour contenir par coulissement des dispositifs de perforation.

4. Un dispositif (1) selon la revendication 3, où lesdits dispositifs porteurs (3) comprennent un élément curviligne (3), dont la première extrémité (3a) est fixée de manière rigide auxdits dispositifs de guidage (2) et dont la deuxième extrémité libre (3b) est fixée rigidement à ladite cible (4).

5. Un dispositif (1) selon la revendication 4, où lesdits moyens d'orientation comprennent un bras mobile (5) et des dispositifs de réglage (8) pour l'orienter spatialement par rapport à la prothèse.

6. Un dispositif (1) selon la revendication 5, où ledit élément curviligne (3) est associé à une extrémité dudit bras mobile (5).

7. Un dispositif (1) selon la revendication 5, où lesdits dispositifs de réglage (8) permettent audit bras (5) de pivoter sur un axe de rotation (Y-Y) et de translater le long de deux directions mutuellement perpendiculaires (Y-Y, Z-Z).

8. Un dispositif (1) selon la revendication 5, où lesdits dispositifs de réglage (8) comprennent des éléments mobiles coopérant mutuellement (8a, 8b, 8c) actionnés par des vis de réglage (11a, 11b, 11c).

9. Un dispositif (1) selon la revendication 1, où lesdits dispositifs porteurs (3) sont insensibles auxdits moyens de détection.

10. Un dispositif (1) selon la revendication 1, où ledit tissu osseux est un fémur et ledit trou passant (10) est un trou distal.
